# DEMANDE DE BREVET EUROPEEN

(11) **EP 2 388 593 A2**
(43) Date de publication de la demande: **23.11.2011**
(21) Numéro de dépôt: 11174571.7
(22) Date de dépôt: 16.10.2007
(51) Int. Cl.: G01N 33/577, C12Q 1/04, C07K 16/12, G01N 33/569

(54) **Procédé de diagnostic in vitro des Staphylococcus aureus producteurs de PVL**

(30) Priorité: 18.10.2006 FR 0654336; 06.02.2007 FR 0753081
(62) Demande divisionnaire de: 07858591.6
(71) Demandeur: bioMérieux, 69280 Marcy L'Etoile (FR); INSTITUT NATIONAL DE LA SANTE ET DE LA RECHERCHE MEDICALE (INSERM), 75654 Paris Cédex 13 (FR); Universite Claude Bernard Lyon 1 (UCBL), 69622 Villeurbanne Cedex (FR)
(72) Inventeur: Badiou, Cédric, 69500 Bron (FR); Etienne, Jérôme, 69300 Caluire (FR); Lina, Gérard, 69007 Lyon (FR); Ratat, Catherine, 69600 Oullins (FR)

(57) **Abrégé**

La présente invention concerne un procédé de diagnostic *in vitro* des *Staphylococcus aureus* productrices de Panton Valentine Leukocidin (PVL) à partir d'un échantillon biologique issu d'un individu susceptible d'être colonisé ou infecté par des *Staphylococcus aureus,* le diagnostic étant mis en oeuvre par détection de PVL par un test immunologique de routine, caractérisé en ce que ledit échantillon biologique est préalablement traité pour dénaturer la PVL.

## Description

La présente invention concerne le domaine des infections liées à la présence de bactéries appartenant au genre Staphylococcus et d'espèce *Staphylococcus aureus* productrices de Panton Valentine Leukocidin (PVL). Plus particulièrement, la présente invention a pour objet un procédé de détermination des *Staphylococcus aureus* producteurs de PVL par un test immunologique de routine dans un échantillon biologique susceptible de contenir des *Staphylococcues aureus.*

Les infections à *Staphylococcus aureus* producteurs de PVL sont essentiellement responsables d'infections communautaires. *Staphylococcus aureus* exprime une grande variété de facteurs de virulence, parmi lesquels la Panton Valentine Leukocidin (PVL), cytotoxine favorisant les dommages tissulaires¹, La PVL est une toxine fréquemment présente dans les souches de *Staphylococcus aureus* résistantes à la méthicilline et transmissible en communauté (« Community acquired methicillin-resistant S. *aureus* » ou CA-MRSA) se propageant dans le monde entier²,

La PVL, ainsi que d'autres leucocidines., est une protéine appartenant à la famille des toxines synergohyménotropiques, Toutes les toxines de cette famille sont constituées de deux composants polypeptidiques. à action synergiques, désignés S et F. La PVL est codée par deux gènes contigus cotranscrits, *lukF-PV et lukS-PV.* La PVL est une exotoxine car elle est excrétée dans le milieu extracellulaire suite à la lyse ou non de la bactérie, contrairement aux endotoxines ou lipopolysaccharides qui, elles, ne sont libérées que lors de la destruction de la bactérie Gram- qui les sécrète.

Les *Staphylococcus aureus* producteurs de PVL, bactéries Gram +, sont associés à des infections humaines spécifiques telles que des infections cutanées, sous-cutanées à type de furoncles, abcès, cellulites et myosite, des infections ostéoarticulaires, ainsi que des pneumonies nécrosantes sévères touchant principalement les enfants et jeunes adultes avec un taux de mortalité d'environ 70%.

La pathogenèse n'est pas complètement connue, mais plusieurs lignes d'évidence suggèrent que la PVL joue un rôle important dans la physiopathologie des infections à *Staphylococcus aureus* producteur de PVL :
i) le fort lien épidémiologique avec les isolats de *S. aureus* synthétisant la PVL, et la présentation clinique de l'infection,
ii) la fréquence élevée de leucopénie, un effet connu de la PVL,
iii) les lésions nécrotiques du tractus respiratoire, ressemblant à la nécrose induite par injection intradermique de PVL chez le lapin,
iv) la présence de PVL dans le poumon ciblant les cellules polymorphonucléaïres,
v) seules les souches productrices de PVL ou la PVL purifiées induisent une pneumonie nécrosante dans les modèles expérimentaux⁷

La détection des *S*. *aureus* producteurs de PVL se fait actuellement par des tests de détection d'acides nucléiques, et notamment par détection des gènes *lukF-PV* et *lukS-PV* par PCR³.

De tels tests ont pour inconvénients d'être indirects car ils ne permettent pas d'affirmer que le gène soit fonctionnel et/ou exprimé, ils sont chers du fait de la nécessité d'un appareillage spécifique, peu rapides et difficiles à mettre en oeuvre. De plus, dans le cadre de l'utilisation d'une PCR, des problèmes de contamination peuvent survenir.

La détection de la PVL a également été mise en oeuvre ponctuellement par immunodiffusion à l'aide d'anticorps polyclonaux⁴, mais cette technique a vite été abandonnée au profit de la détection de gènes car elle est très peu aisée, difficilement standardisable, de sorte qu'elle ne constitue pas un bon outil pour être utilisée en routine.

En raison de la gravité de certaines infections dues aux *Staphylococcus aureus* producteurs de PVL, il devient urgent d'avoir à disposition un test rapide et simple à mettre en oeuvre qui pallierait les inconvénients des tests actuellement utilisés pour la détection des *S*. *aureus* producteurs de PVL.

La demande de brevet EP 597 110 A décrit que la détection des MRSA peut être mise en oeuvre par des tests immunologique de routine en utilisant des anticorps monoclonaux anti-PVL. Toutefois, bien que ces tests permettent de pallier les inconvénients ci-dessus, la spécificité de détection n'est pas suffisante.

Contre toute attente, les Demanderesses ont maintenant mis en évidence que la spécificité de la détection des *S. aureus* producteurs de PVL par un test immunologique de routine pouvait être améliorée en effectuant un traitement préalable de l'échantillon biologique pour dénaturer la PVL, alors qu'il est connu de l'Homme du Métier que, comme toute exotoxine, la PVL est sensible à des agents pysico-chimiques comme la température⁵.

ainsi, la présente invention a pour objet un procédé de diagnostic *in vitro* des *Slaphylococcus aureus* producteurs de Panton Valentine Leukocidin (PVL) à partir d'un échantillon biologique issu d'un individu susceptible d'être colonisé ou infecté par des *Staphylococcus azereus,* le diagnostic étant mis en oeuvre par détection de PVL par un test immunologique de routine, caractérisé en ce que ledit échantillon biologique est préalablement traité pour dénaturer la PVL.

Par *Staphylococcus aureus* producteurs de PVL, on entend toutes les souches de *Staphylococcus aureus* capables de produire de la PVL, à savoir tant les souches sensibles à la méthicilline (également appelées MSSA), que celles résistantes à la méthicilline (également appelées MRSA).

Par individu susceptible d'être colonisé par des *Staphylococcus aureus,* on entend les individus à risque qui peuvent être porteurs sains de cette bactérie, tels que le personnel hospitalier. Par individu susceptible d'être infecté par des *Staphylococcus aureus,* on entend les patients présentant les symptômes d'une infection à *Staphylococcus aureus.*

Par infection à *Staphylococcus aureus,* on entend toute infection provoquée par la présence de cette bactérie dans un organisme, que ces infections soient à *Staphylococcus aureus* producteurs de PVL ou à *Staphylococcus aureus* n'ayant pas une telle capacité. A titre d'exemple de telles infections, on peut citer les infections suppuratives de la peau et des tissus mous, les infections du tractus respiratoire, les infections du système nerveux central, les infections urinaires, les infections endovasculaires et valvulaires cardiaques et les infections musculaires et osseuses. De telles infections et les symptômes associés sont largement connues de l'homme du métier et sont décrits notamment dans le Précis de Bactériologie Clinique⁵.

Par échantillon biologique issu d'un individu susceptible d'être colonisé ou infecté par des *Staphylococcus aureus,* on entend tout échantillon susceptible de contenir ces bactéries ou bien la PVL excrétée, tel que pus, prélèvement respiratoire, prélèvement nasal, urine, hémoculture.

L'échantillon biologique mis en oeuvre dans le procédé de l'invention peut être l'échantillon non modifié, ou bien il peut consister en une culture des bactéries issue de cet échantillon. La culture peut être mise en oeuvre sur un milieu solide, tel qu'une boite de Pétri. ou dans un bouillon de culture, étant entendu que le bouillon de culture peut être inoculé par ledit échantillon ou bien par des colonies de *Staphylococcus aureus* préalablement isolées dudit échantillon biologique par des méthodes bien connues de l'homme du métier, telles que par ensemencement sur boîte de Pétri. Le test immunologique sera alors mis en oeuvre soit directement sur le milieu solide, soit dans le bouillon de culture. De façon générale, la mise en culture des bactéries d'intérêt ou dudit échantillon dure environ 24h. On peut noter que, quelque soit la culture, le milieu de culture comprend un agent favorisant la production de PVL, tel que le milieu CCY (Casein hydrolysate and yeast extract médium), avec ou sans utilisation de molécules permettant une plus grande production de PVL, telle que l'oxacilline et la bactracine à dose sub-inhibitrice.

Par test immunologique, on entend tout test immunologique utilisant un partenaire de liaison capable de se lier spécifiquement à la PVL, que ce soit la fraction LukF de la PVL, la fraction LukS de la PVL, ou bien les deux fractions simultanément.

Par test immunologique de routine, on entend tout test immunologique largement utilisé dans la routine d'un laboratoire. A titre d'exemple de tests immunologiques de routine, on peut citer les tests sandwich de type ELISA ou immunochromatographique (également appelé lateral flow), et les tests d'agglutination de particules, par exemple de particules de polystyrène. Tous ces tests sont largement connus de l'homme du métier. Le test sandwich peut être réalisé en une ou plusieurs étapes, c'est-à-dire sans étape de lavage ou avec une ou plusieurs étapes de lavage.

A titre d'exemple de partenaire de liaison spécifiques de PVL, on peut citer les anticorps, les fractions d'anticorps, les récepteurs, les mimotopes et toute autre molécule capable de se lier à la PVL.

Les anticorps partenaires de liaison sont par exemple soit des anticorps polyclonaux, soit des anticorps monoclonaux.

Les anticorps polyclonaux peuvent être obtenus par immunisation d'un animal avec de la PVL, une fraction de la PVL ou un peptide de la PVL, suivie de la récupération du sérum dudit animal. Les anticorps peuvent être utilisés purifiés ou non dans le procédé de l'invention. La purification des anticorps polyclonaux peut être mise en oeuvre par exemple par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment la PVL.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vïtro*) avec de la PVL, une traction de la PVL ou un peptide de la PVL, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal reconnaissant spécifiquement la PVL. Les anticorps peuvent être utilisés purifiés ou non dans le procédé de l'invention. Les anticorps monoclonaux peuvent être purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

Par fragment d'anticorps utiles aux fins de l'invention, on entend les fragments de type F(ab')2, Fab, Fab', scFv d'un anticorps natif, lesquels ont conservé la capacité de liaison spécifique à la PVL.

Le procédé de l'invention est mis en oeuvre de préférence avec les caractéristiques suivantes, prises seules ou en association :
- le test immunologique de routine est une méthode sandwich,
- il met en oeuvre des partenaire(s) de liaison spécifique de LukS- PV ou LukF-PV,
- il met en oeuvre des fragments d'anticorps et notamment le fragment F(ab')2,

Dans le cadre d'un test sandwich mettant en oeuvre deux partenaires de liaison spécifique à la PVL, on peut utiliser par exemple deux anticorps monoclonaux, deux anticorps polyclonaux, ou leur fragments, ou bien un anticorps monoclonal et un anticorps polyclonal, ou leurs fragments. Dans le cadre d'un test d'agglutination de particules, on utilise un seul partenaire de liaison spécifique à la PVL, par exemple soit un anticorps monoclonal, soit un anticorps polyclonal, sous forme de fragment ou non.

Selon un mode de réalisation particulier, le test immunologique met en oeuvre au moins un anticorps monoclonal anti-PVL.

Dans le cadre d'un test d'agglutination de particules, le partenaire de liaison spécifique de la PVL est utilisé en capture. Dans le cadre d'un test sandwich, ils sont utilisés en capture et en détection.

Lorsque le partenaire de liaison est utilisé comme réactif de détection, il est marqué pour la révélation de la liaison PVL/partenaire de liaison.

Par marquage des partenaires de liaison, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la peroxydase de raifort, la phosphatase alcaline, la α-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que l'alexa ou les phycocyanines.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps. peptide/anticorps, sucre/lectine, polynucléotide/complémentaire du polynucléotide, Dans ce cas, c'est le ligand qui porte le partenaire de liaison. L'anti-ligand peut être détectable directement par les marqueurs décrits au paragraphe précédent ou être lui-même détectable par un ligand/anti-lisand.

Ces systèmes indirects de détection peuvent conduire, dans certaines conditions, a une amplification du signal. Cette technique d'amplification du signal est bien connue de l'homme du métier, et l'on pourra se reporter à l'article dans J. Histochemo Cytochem6, 45 : 481-491, 1997.

Selon le type de marquage utilisé, l'homme du métier ajoutera des réactifs permettant la visualisation du marquage.

Dans le cas des méthodes dites de compétition, la PVL est marquée comme décrit précédemment pour le partenaire de liaison.

Le partenaire de liaison spécifique de la PVI, s'il est utilisé en capture, est alors immobilisé directement ou indirectement sur une phase solide par des procédés connus de l'homme du métier.

Le traitement préalable de l'échantillon utilisé dans le procédé de l'invention peut être tout traitement de dénaturation de protéines largement connu de l'homme du métier. Ce traitement peut être mis en oeuvre par exemple par modification du pH, par utilisation d'agents dénaturants chimiques, tels que urée, sodium dodécylsulfate (SDS), ions guanidinium, ou bien par un chauffage de l'échantillon dans lequel se trouve la protéine à dénaturer.

Selon un mode de réalisation, le traitement préalable dudit échantillon consiste en un chauffage à une température comprise entre 60 et 100 °C en une durée d'au moins 10 min. De préférence, le chauffage est effectué à une température comprise entre 80 et 100 °C, de préférence encore entre 90 et 100°C.

Bien entendu, le traitement préalable de l'échantillon sera appliqué à l'échantillon lui-même lorsque le test immunologique de routine est mis en oeuvre dans ledit échantillon, ou bien il sera appliqué à la culture lorsque le test immunologique est mis en oeuvre dans ladite culture.

Le procédé de l'invention peut également comprendre une étape supplémentaire de vérification de la présence de *Staphylococcus aureus* dans ledit échantillon biologique, cette étape pouvant être mise en oeuvre de façon préalable ou concomitante. Les procédés de détection de ces bactéries sont connues de l'homme du métier et on peut citer, à titre d'exemple, l'utilisation de milieux contenant un agent chromogène spécifique de cette bactérie, comme décrit par exemple dans la demande de brevet WO02079486 déposée par l'une des Demanderesses.

De même, le procédé de l'invention peut comprendre une étape supplémentaire consistant à déterminer si les *Staphylococcus aureus* présents dans ledit échantillon biologique sont des bactéries résistantes à la méthicilline (MRSA) ou sensibles à la méthicilline (MSSA). ce qui constitue un mode de réalisation particulier de l'invention.

Cette étape de détermination de la sensibilité à la méthicilline (MRSA ou MSSA) peut être mise en oeuvre par des procédés largement connus de l'homme du métier tels que des tests immunologiques utilisant par exemple un partenaire de liaison spécifique à la protéine PBP2', qui est une protéine exprimée uniquement par les MRSA, des tests de détection d'acide nucléique ou bien des tests microbiologiques, par exemple mettant en oeuvre un milieu contenant un antibiotique tel que l'oxacilline ou une céphalosporine, par exemple la céfoxitine.

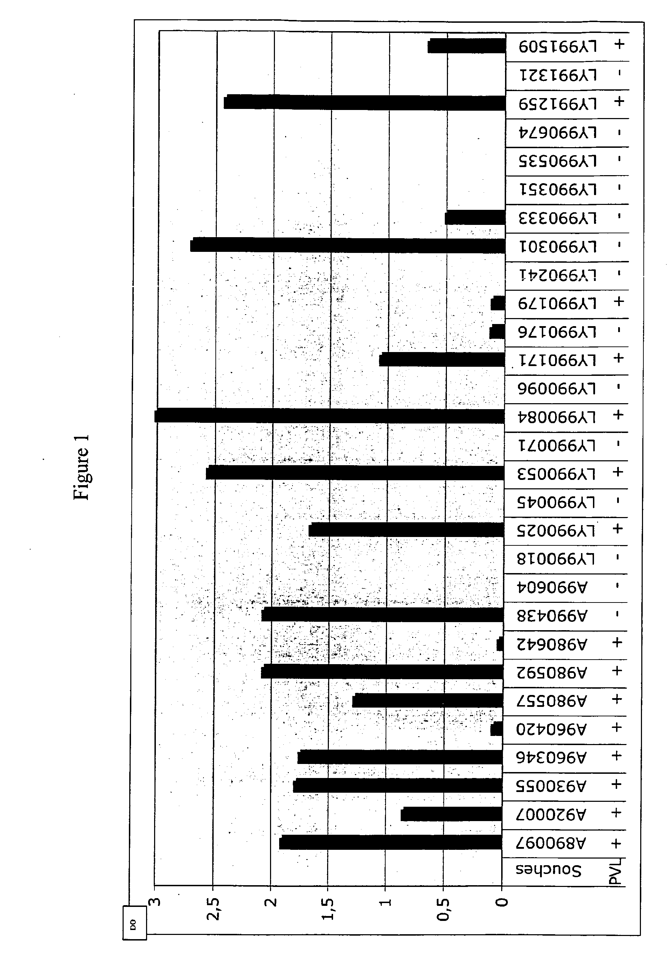

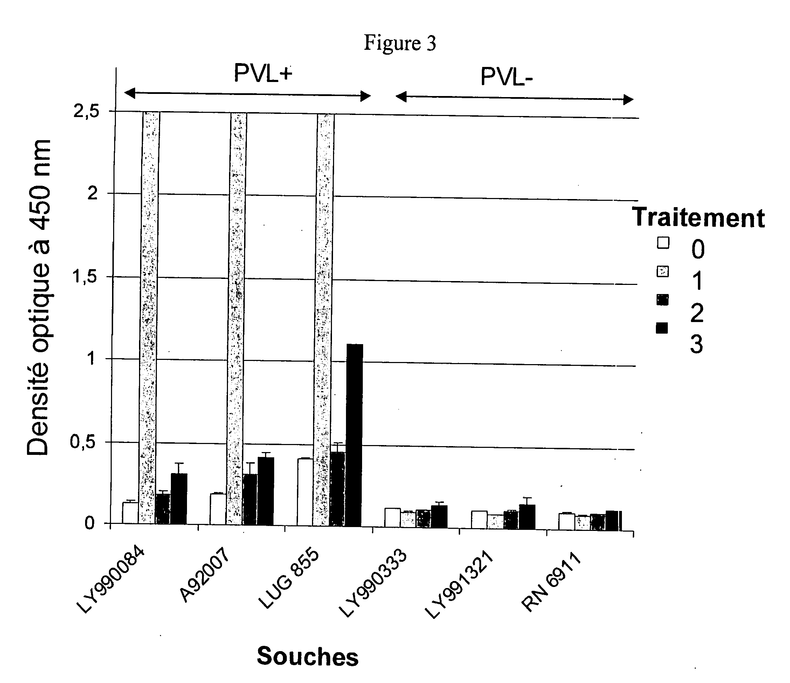

L'invention sera mieux comprise à l'aide des exemples suivants donnés à titre illustratif et non limitatif ainsi qu'à l'aide des figures 1 et 3 annexées, sur lesquelles :
- la figure 1 est un graphe donnant les résultats d'un test ELISA (DO de chaque souche) pour la détection de PVL dans des échantillons biologiques contenant des souches de *Staphylococcus aureus* productrices de PVL (PVL+) ou non productrices de PVL (PVL-),

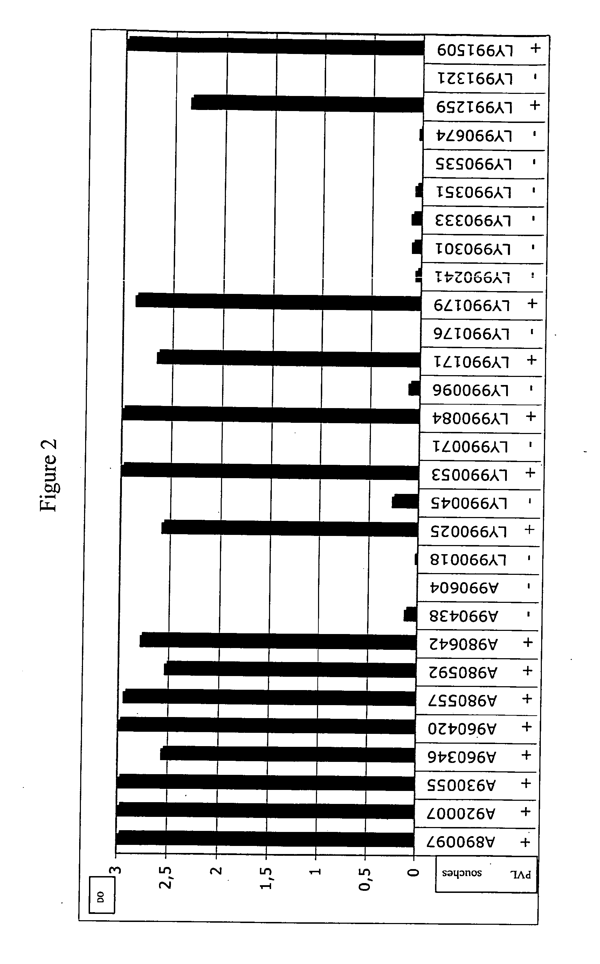

- la figure 2 est un graphe donnant les résultats d'un test ELISA (DO de chaque souche) pour la détection de PVL dans des échantillons biologiques contenant des souches de *Staphylococcus aureus* productrices de PVL (PVL+) ou non productrices de PVL (PVL-), ledit échantillon ayant été préalablement traité par chauffage pour dénaturer la PVL, et
- la figure 3 est un graphe donnant les résultats d'un test ELISA (DO de chaque souche) pour la détection de PVL dans des échantillons biologiques contenant des souches de *Staphylococcus aureus* productrices de PVL (LY990084, A92007, LUG855) ou non productrices de PVL (LY990333, LY991321, RN6911), ledit échantillon ayant été préalablement traité par chauffage (traitement 1), par un agent de dénaturation chimique (traitement 2), par un agent de dénaturation et par chauffage (traitement 3), l'état 0 correspondant à aucun traitement préalable de l'échantillon.

### Exemple 1 : Préparation des anticorps anti-PVL

### 1. Production de PVL recombinante

On a produit les protéines LukS His-Tag et LukF His-Tag de séquence respective SEQ ID N°1 et SEQ ID N°2 en utilisant le vecteur pIVEX 2.4d (Roche) transformé dans une souche de *Escherichia colï* BL21star(DE3) pLys (Invitrogen) lors d'une culture de 2 litres à 37°C avec agitation, induction 3h à 37°C par 1mM IPTG (isopropyl-beta-D-thiogalactopyranoside, eurobio), puis culture pendant 5 heures supplémentaires. La culture est répartie dans 6 tubes différents.

Chaque culot cellulaire est récupéré par centrifugation à 4000g de 20 min et élimination du surnageant. La purification des protéines recombinantes est réalisée à l'aide du kit The QIAexpressionist selon les recommandations du fournisseur (Qiagen). Le culot est lysé par 20 ml d'une solution de lyse non dénaturante (Qiagen) à 4°C, puis conservé une nuit à -80°C. Après décongélation, la lyse est poursuivie par un traitement par lysozyme (Eurobio) 1 mg/ml 1h à 4°C avant une sonication par Vibracell (Bioblock) 100 Watt. 2 min en cycles de 6 s.

La solution est centrifugée à 10 000 g pendant 30 min à 4°C. Le surnageant est mis en contact avec 5 ml d'agarose-NiNTA (Qiagen) pendant 3h à 4°C avec une agitation circulaire douce. L'agarose est ensuite mis dans une colonne de 20 ml (Biorad) La colonne est lavée par 200 ml de tampon de lavage Wash buffer (Qiagen), puis éluée par de l'imidazole 250 mM à titre de tampon d'élution. Cette purification est complétée par une purification sur colonne échangeuse d'ion monoSP (Amersham).

Les protéines recombinantes sont concentrées sur Centricon (Vivaspin). dialysées contre un tampon 50 mM MES (acide 2-(N-morpholino)éthanesulfonique), puis élue par un gradient de NaCl (0 à 1M). Les protéines sont ensuite dialysées contre de l'eau stérile apyrogène.

### 2. Production d'anticorps monoclonaux

On a obtenu les anticorps monoclonaux suivants :
- anti-LukF : 10D1A10, 16A10A3. 6H10 E5 et
- anti-LukS : 2H2H12. 3D9D12. 7C1F9, 7F8D7, 18A4E10 comme suit :

### Anticorps monoclonaux anti-Luk F : 10D1A10, 16A10A3 , 6H10E5.

Les souris ont été immunisées selon le protocole suivant : au jour J0 injection intrapéritonale de 10 µg de protéine recombinante Luk F en présence d'adjuvant de Freund complet. Aux jours J14, J28, nouvelle injection intrapéritonéale de la même quantité de protéine recombinante Luk F en présence d'adjuvant de Freund imcomplet. Quatre jours avant la fusion faire une injection intraveineuse de 50 µg d'antigène Luk F dilué en eau physiologique.

1600 surnageants ont été criblés par technique d'ELISA indirect. Les plaques ont été « coatées » avec 100 µl d'antigène (Protéine recombinante Luk F) à 1µg/ml en tampon PBS, pH 7.2. Les plaques « coatées » ont été incubées une nuit à la température de 18-22°C. Les plaques ont été saturées avec 200µl de PBS-lait 1% et soumises à incubation 1 heure à 37°+/-2°C. 100 µl de surnageants ou de liquide d'ascites dilués en tampon PBS-tween 20, 0.05% ont été ajoutés et les plaques ont été incubées 1 heure à 37°+/-2°C. 100 µl d'anticorps polyclonal de chèvre anti-Ig(H+L) de souris conjugué à la phosphatase alcaline (Jackson Immunoresearch réf:115-055-062), dilué en tampon PBS-BSA 1% au 1/2000, ont été ajoutés et les plaques ont ensuite été incubées 1 heure à 37°+/-2°C, 100µl de PNPP (bioMérieux réf 60002990) à la concentration de 2mg/ml dans de la DEA-HCL (bioMérieux réf 60002989), pH=9,8, ont été ajoutés. Les plaques ont été soumises à incubation pendant 30 minutes à 37°+/- 2°C. La réaction a été bloquée par addition de 100 µl de NaOH, 1N. Trois lavages sont effectués entre chaque étape avec 300µl de PBS-tween 20, 0,05%. Un lavage supplémentaire en eau distillée est effectué avant d'ajouter le PNPP.

72 surnageants se sont révélés positifs en ELISA indirect avec une DO > 0.6. Après les tests de spécificité les trois anticorps précités sont produits.

### Anticorps monoclonoux anti-Luk S: 2H2H12, 3D9D12, 7C1F9, 7F8D7, 18A4E10

Les souris ont été immunisées selon le protocole suivant : au jour J0 injection intrapéritonale de 10 µg de protéine recombinante Luk S en présence d'adjuvant de Freund complet. Aux jours J14. J28 nouvelle injection intrapéritonéale de la même quantité de protéine recombinante luK S en présence d'adjuvant de Freund imcomplet. Quatre jours avant la fusion faire une injection intraveineuse de 50 µg protéine recombinante Luk S dilué en eau physiologique.

1800 surnageants ont été criblés par technique d'ELISA indirect. Les plaques ont été « coatées » avec 100 µl protéine recombinante Luk S à 1 µg/ml en tampon PBS, pH 7.2. Les plaques « coatées » ont été incubées une nuit à la température de 18-22°C. Les plaques ont été saturées avec 200µl de PBS-lait 1% et soumises à incubation 1 heure à 37°+/-2°C. 100 µl de surnageants ou de liquide d'ascites dilués en tampon PBS-tween 20, 0.05% ont été ajoutés et les plaques ont été incubées 1 heure à 37°+/- 2°C. 100 µl d'anticorps polyclonal de chèvre anti-Ig(H+L) de souris conjugué à la phosphatase alcaline (Jackson Immunoresearch réf-1 15-055-062), dilué en tampon PBS-BSA 1% au 1/2000, ont été ajoutés et les plaques ont ensuite été incubées 1 heure à 37°+/-2°C. 100µl de PNPP (Biomérieux réf 60002990) à la concentration de 2mg/ml dans de la DEA-HCL (Biomérieux réf 60002989), pH=9,8, ont été ajoutés. Les plaques ont été soumises à incubation pendant 30 minutes à 37°+/-2°C. La réaction a été bloquée par addition de 100 µl de NaOH, 1N. Trois lavages sont effectués entre chaque étape avec 300µl de PBS-tween 20, 0.05%. Un lavage supplémentaire en eau distillée est effectué avant d'ajouter le PNPP.

51 surnageants se sont révélés positifs en ELISA indirect avec une DO > 0.6. Après les tests de spécificité les cinq anticorps précités sont produits.

### 3. Production d'anticorps polyclonaux

### 3.1. Production d'anticorps polyclonaux anti-Luk S

On a obtenu les anticorps polyclonaux anti-LukS n° 173/89 et 176/89 comme suit :

Au jour J0, des lapins (New Zealand Wite) ont reçu une injection intradermique de 200 µg de peptide synthétique de LukS-PV de séquence CSGHDPNLFVGYKPYSQN (SEQ ID N°3) couplé en N terminal avec la KLH (Keyhole Limpet Hemocyanin) (Agro-Bio). Aux jours J14, J28, J42 et J81, les lapins ont reçu une nouvelle injection sous-cutanée de la même quantité de peptide synthétique couplé à la KLH. Le sérum des animaux est prélevé à J0, J49 et J89. Les sérums de lapins prélevés à J89 sont purifiés par chromatographie d'affinité sur ledit peptide synthétique. Pour cela, ledit peptide est couplé sur une colonne de 1 ml Hi-Trap sépharose selon les recommandations du fabriquant (Amersham-Pharmacia). Le sérum est dilués au demi en tampon PBS, pH 7,4, puis injecté à raison de 1ml/min sur la colonne. Après lavage avec du PBS, pH 7.4, puis élution par 50 mM de mélange glycine/HCl, pH 3, les fractions éluées sont immédiatement neutralisées, puis dialysées contre du PBS 0,15M, pH 7.4,

### 3.2. Production d'anticorps polyclonaux anti-Luk F

On a obtenu les anticorps polyclonaux anti-LukF comme suit :

Au jour J0, des lapins (New Zealand Wite) ont reçu une injection intradermique de 200 µg de peptide synthétique de LukF-PV de séquence CNFNWIGNNYKDENRATHTS (SEQ ID N°4) couplé en N terminal avec la KLH (Keyhole Limpet Hemocyanin) (Agro-Bio). Aux jours J14, J28, J42 et J81, les lapins ont reçu une nouvelle injection sous-cutanée de la même quantité de peptide synthétique couplé à la KLH. Le sérum des animaux est prélevé à J0, J49 et J89. Les sérums de lapins prélevés à J89 sont purifiés par chromatographie d'affinité sur ledit peptide synthétique, Pour cela, ledit peptide est couplé sur une colonne de 1 ml Hi-Trap sépharose selon les recommandations du fabriquant (Amersham-Pharmacia). Le sérum est dilué au demi en tampon PBS, pH 7,4, puis injecté à raison de 1ml/min sur la colonne. Après lavage avec du PBS, pH 7,4, puis élution par 50 mM de mélange glycine/HCL pH 3, les fractions éluées sont immédiatement neutralisées, puis dialysées contre du PBS 0,15M, pH 7.4

### 4. Production et couplage de fragments d'anticorps

On a procédé à la digestion des anticorps polyclonaux anti-LukS 173/89 et 176/89 avec de la pepsine fixée sur agarose en 1h30 à pH 3.5 à 37°C pour éliminer leur fragment Fc et ainsi obtenir les fragments F(ab°)2.

On a marqué ces fragments à la peroxydase comme suit : les fragments F(ab')2 sont dialyses contre du tampon carbonate pH 9,6 et couplés à la peroxydase (Roche) préalablement oxydée par NaIO₄, 2 heures à 18-25°C avec un ratio : 1 mole de F(ab')2 pour 2 moles de peroxydase. Le couplage est ensuite bloqué par du NaBH₄ 1 heure à 2-8°C puis le produit est dialysé contre du tampon PBS avec conservateurs.

On les a ensuite marqués à la biotine comme suit : les fragments F(ab')2 sont dialysés contre du tampon carbonate pH 8,3 et couplés à la biotine-NHS (Roche) 1 heures à 18-25°C avec un ratio de 1 mole de F(ab')2 pour 10 moles de biotine. Le couplage est bloqué par de la lysine 20 minutes à 18-25°C, puis le produit est dialysé contre du PBS + azide.

### Exemple 2 : Diagnostic des souches de Staphylococcus aureus productrices de PVL

### 1. Préparation de l'échantillon biologique

Les souches cliniques de *Staphylococcus aureus* sont précultivées sur gélose GP (Difco : peptone 10 g/l, extrait de levure 5 g/l, agar 17 g/l ; Sigma : NaCl 5 g/l, glucose 1 g/l). Après 18h à 37°C, la pureté est vérifiée avant ensemensement de quelques colonies (environ 10⁷ UFC/ml) dans 5 ml de milieu CCY (Difco : extrait de levure 30 g/l. acide casaminé 20 g/l ; Sigma : Na₂HPO₄ 2H₂O 3,11 g/l, KH₂PO₄ 0,41 g/l, acide pyruvique 23 g/l) dans un flacon Erlenmeyer en verre de 25 ml. Après culture de 18 h à 37°C avec agitation, les surnageants de culture sont récupérés après centrifugation (8 000 g, 4°C, 10 min), puis sont conservés pour une courte durée à 4°C ou à -20°C avant utilisation.

Chaque souche a reçu un identifiant (A ou LY complété de 6 numéros) et est désignée « + » si elle produit de la PVL ou « - » si elle n'en produit pas

### 2. Test ELISA

Les puits de plaques ELISA (Greiner, 100 µl/puits) sont recouverts d'anticorps anti-LukS 18A4E10 par incubation avec une solution de cet anticorps dilué dans du PBS (PBS Sigma, pH 7,4 ; 0,1% d'azoture de sodium) à 10 µg/ml pendant 18 h à température ambiante (environ 22°C). Après 5 lavages en PBS Tween 20 (appareil ImmunoWash 1575 Biorad), la plaque est saturée avec une solution de PBS Tween (0.05%) -lait 10% - 0,5% BSA (Sigma) pendant 2 h à température ambiante (150 µl/puits).

Après 5 lavages avec du PBS Tween (0,05%), les surnageants de culture sont incubés 75 min à 37°C.

Après 5 lavages avec du PBS Tween (0.05%), la solution d'anticorps de lapin 176-89, fragment F(ab'2) marqué à la peroxydase, dilué en PBS-T lait 5% au 1/100, est incubée 75 min à 37°C. Après 5 lavages avec du PBS-Tween, 75 µl de solution de IMB (substrat 3,3',5,5'-tétraméthylbenzine, Sigma) sont ajoutés et la plaque est incubée 30 min à l'obscurité La réaction est stoppée par ajout de 75 µl de H₂SO₄ 1N. La lecture des plaques (DO) est réalisée à 450 nm grâce au lecteur MicroPlaque Reader 680 (Biorad).

### 3. Résultats

Les résultats sont présentés sur la figure 1 qui est un graphe donnant la DO de chaque souche.

Les resultats montrent que, parmi les 15 souches PVL+, 12 souches sont bien détectées et que, parmi les 14 souches PVL-, on observe 3 résultats faux positifs.

Ces résultats montrent clairement qu'on peut différencier les souches PVL+ des souches PVL- à l'aide d'un test immunologique de routine, mais que ce dernier manque de spécificité

### Exemple 3 : Diagnostic des souches de Staphylococcus aureus productrices de PVL

### après traitement préalable de l'échantillon biologique par chauffage

### 1. Traitement de l'échantillon

On a procéde au traitement par chauffage à 95°C des échantillons biologiques, tels que préparés dans l'exemple 2, pendant des temps variant de 10 à 30 min et on a déterminé leur DO selon le protocole décrit dans l'exemple 2.

1 es résultats de DO sont donnés dans le tableau 1 ci-après qui montre qu'on obtient une dénaturation de la PVL dès 10 min de traitement.

**tableau 1**

| N de Souches | PVL | Sans chauffage | Chauffage 10 min | Chauffage 20 min | Chauffage 30 min |
|---|---|---|---|---|---|
| LY990179 | - | 0.839 | 2.126 | 2.76 | >3 |
| A960420 | - | 1.73 | 2.844 | >3 | >3 |
| A990438 | - | 1.545 | 0,852 | 0,25 | 0 |
| A980642 | - | 0,458 | 1,364 | 2,325 | >3 |
| LY990301 | - | 2,123 | 0,105 | 0 | 0 |
| HT20020275 | - | 0,729 | 2,269 | >3 | >3 |
| LY990333 | - | 0,615 | 0202 | 0 | 0 |
| HT20040540 | - | 1,178 | >3 | >3 | >3 |

### 2. Test ELISA

On a répété le mode opératoire décrit dans l'exemple 2, point 2 ci-dessus, à ceci près qu'on a utilisé les surnageants de culture de 29 souches (15 PVL+ et 14 PVL-) préalablement traités à 95°C pendant 1 h.

### 3. Résultats

Les résultats sont présentés sur la figure 2 qui est un graphe donnant la DO de chaque souche.

Les résultats montrent que toutes les souches PVL+ sont identifiées par le procédé de l'invention avec un niveau de DO suffisamment élevé pour faire une distinction avec les souches PVL-.

Le traitement préalable permet donc d'améliorer la spécificité du procédé de l'invention.

### Exemple 4 : Diagnostic des souches de Staphylococcus aureus productrices de PVL après traitement préalable de l'échantillon biologiques par plusieurs types de traitement de dénaturation

### 1. Traitement de l'échantillon

On a utilisé trois souches cliniques PVL+ (LY990084, A92007, LUG855) et trois souches cliniques PVL- (LY990333, LY991321, RN6911), auxquelles on a fait subir les traitements suivants :
- absence de traitement (0),
- dénaturation par chauffage à 95°C pendant 1 h (1),
- dénaturation par l'acide KH₂PO₄ 0,1M final pendant 30 min sur vortex, puis neutralisation par ajout de NaOH (2).
- dénaturation mixte par l'acide KH₂PO₄ 0,1 M final pendant 10 min sur vortex, puis ébullition 10 min à 95°C, suivie d'une neutralisation par NaOH (3).

### 2. Test ELISA

On a répété le mode opératoire décrit dans l'exemple 2, à ceci près qu'on a utilisé les souches prétraitées selon le point 1 ci-dessus et une solution d'anticorps de lapin 176-89, fragment F(ab'2) marqué à la peroxydase, dilué en PBS-Tween lait 5% au 1/1000.

### 3. Résultats

Les résultats sont présentés sur la figure 3 qui est un graphe donnant la DO de chaque souche.

Les résultats montrent que toutes les souches PVL- ont des valeurs de DO comprises entre 0.09 et 0,181, quel que soit le traitement de l'échantillon.

Pour les souches PVL+, les valeurs de DO sont plus élevées que celles des souches PVL- après traitement. L'augmentation de DO obtenue par traitement chimique, par traitement thermique et par combinaison des deux est statistiquement significative (p=0,024, p<0.001. p=0,018, respectivement), même si le traitement thermique apparaît dans ces conditions le plus performant.

Le traitement préalable permet donc d'améliorer la spécificité d'un test immunologique de routine.

### Exemple 5 : Mise en évidence de la détection précoce des bactéries Staphylococcus aureus productrices de PVL par l'utilisation du procédé de l'invention

### 1. Préparation de l'échantillon biologique

On a précultivé quatre souches cliniques de *S aureus* (2 souches PVL+ et 2 souches PVL-) selon le mode opératoire décrit dans l'exemple 2. Les colonies sont utilisées pour réaliser un ensemencement riche de 5 ml de milieu CCY dans un flacon Erlenmeyer en verre de 25 ml. Le milieu de culture est prélevé immédiatement après inoculation, puis successivement à 1h, 2h, 3h et 18h après incubation à 37°C avec agitation. Les surnageants de culture sont récupérés après centrifugation (8 000 g, 4°C, 10 min), puis sont conservés pour une courte durée à 4°C ou à -20°C avant utilisation. Les échantillons sont traités par chauffage comme décrit dans l'exemple 3.

### 2. Test ELISA

On a répété le mode opératoire décrit dans l'exemple 2, point 2 ci-dessus, à ceci près qu'on a utilisé les surnageants de culture des souches préalablement traités comme décrit dans le point 1 ci-dessus.

### 3. Résultats

Les résultats de DO sont donnés dans le tableau 2 qui montre que la PVL peut être détectée très rapidement avec le procédé de l'invention (détection nette dès 2h de culture).

**Tableau 2**

| | | Temps de culture | | | | |
|---|---|---|---|---|---|---|
| Nde souches | | T0 | T1h | T2h | T3h | T18h |
| A89 0097 | PVL + | 0.161 | 1.116 | >3 | >3 | >3 |
| A92 0007 | PVL + | 0.083 | 0.975 | 2.57 | >3 | >3 |
| LY99 0301 | PVL- | 0 | 0 | 0 | 0 | 0 |
| LY99 0333 | PVL- | 0 | 0 | 0 | 0 | 0 |

### Exemple 6: Mise en évidence de la détection précoce de PVL dans les prélèvements biologiques par l'utilisation du procédé de l'invention

### 1. Préparation de l'échantillon biologique

Les prélèvements cliniques sont des aspirations broncho-pulmonaires, des liquides broncho-alveolaires et des pus d'abcès cutanées ou de suppuration profonde de patients présentant des infections à *S aureus* avec soit des souches PVL+, soit des couches PVI - Ces prélèvements on été conservés à -20°C avant l'analyse par l'utilisation du procédé de l'invention. Après décongélation à température ambiante, les prélevements sont homogénéisés par agitation au Vortex pendant 15 min. Après centrifugation (10 min, à 4000tr/min à 15°C), le surnageant est transféré dans un tube Eppendorf, puis dénaturé 1h à 95°C.

Pour les échantillons très visqueux, les échantillons sont d'abords dilués au demi avec du PBS pH 7.4 puis agités au Vortex pendant 15 min. Après centrifugation (10 min. à 4000tr min à 15°C), le surnageant est transféré dans un tube Eppendorf, puis dénaturé 1h à 95°C.

En cas de surnageant visqueux, celui-ci est dilué au demi avec du n-Heptane (Merck), agité à nouveau 10 min au Vortex. Après centrifugation (10 min, à 4000tr/min a 15°C), la phase supérieure correspondant au n-Heptane est totalement éliminée et la phase inférieure transférée dans un autre tube Eppendorf pour être dénaturée 1h à 95°C,

### 2. Test ELISA

On a répété le mode opératoire décrit dans l'exemple 2, point 2 ci-dessus, à ceci près que l'on utilise les prélèvements biologiques traités comme décrit dans le point 1 ci-dessus.

### 3. Resultats

Les résultats de dosage de la PVL sont donnés dans les tableaux 3 et 4 qui montrent que la PVL peut être détectée directement dans les prélèvements biologiques, quelle que soit la sensibilité de la souche à la méthicilline, et qu'il n'existe aucun faux positif (aucun prélèvement avant des souches PVL- n'est détecté comme contenant de la PVI). Par ailleurs, le prétraitement du prélèvement par le n-Heptane n'altère pas la détection de la PVL.

**Tableau 3**

| Prélèvement | Type de prélèvement | Prétraitement du prélèvement | Souche* | Quantité de PVL (µg/ml) |
|---|---|---|---|---|
| 17 | Lavage Broncho Alvéolaire | non | PVL+ | <0,05 |
| 13 | Aspiration Bronchique | non | PVL- | <0,05 |
| 18 | Nlini Lavage Broncho Alvéolaire | non | PVL- | <0,05 |
| 22 | Aspiration Bronchique | non | PVL- | <0,05 |
| 11 | Aspiration Bronchique | non | PVL- | <0,05 |
| 8 | Aspiration Bronchique | PBS - n-Heptane | PVL- | <0,05 |
| 16 | Aspiration Bronchique | non | PVL- | <0,05 |
| 10 | Aspiration Bronchique | non | PVL- | <0,05 |
| 19 | Crachat | non | PVL- | <0,05 |
| 14 | Aspiration Bronchique | non | PVL- | PVL- |
| 15 | Aspiration Bronchique | non | PVL- | <0.05 |
| 12 | Aspiration Bronchique | non | PVL- | <0,05 |
| 7 | Aspiration Bronchique | non | PVL- | <0,05 |
| 5 | Aspiration Bronchique | non | PVL- | <0.05 |
| 23 | Liquide articulaire | non | PVL- | <0.05 |
| 20 | Liquide péritonéal | non | PVL- | <0.05 |
| 4 | liquide de drain | non | PVL + | <0.05 |
| 2 | Abcès | non | PVL+ | 1 |
| 3 | Abcès | non | PVL- | <0.05 |
| 1 | Liquide de drain | non | PVL- | <0,05 |
| 5 | Aspiration Bronchique | PBS | PVL+ | 1,2 |
| 5 | Aspiration Bronchique | PBS - n-Heptane | PVL + | 1,2 |
| 6 | Aspiration Bronchique | PBS + n-Heptane | PVL + | 1,2 |

| | | | | |
|---|---|---|---|---|
| ^{*} L'appartenance à PVL- ou PVL+ a été mise en oeuvre par la détection de la présence par PCR des gènes lukS-PV et lukF-PV codant la PVL au sein des isolats de *S*. *aureus* selon les méthodes décrites par Vandenesch et al.² | | | | |

**Tableau 4**

| | Echantillon | Souche* | Concentration de Luk S-PV (µg/ml) | Détection gène mecA** |
|---|---|---|---|---|
| Pneumonia | Lavage Broncho Alvéolaire | PVL+ | 0,99 | 0 |
| | Lavage Broncho Alvéolaire | PVL+ | 1,07 | 0 |
| | Lavage Broncho Alvéolaire | PVL+ | 20,18 | 0 |
| | Crachat | PVL- | 0,00 | 1 |
| | Crachat | PVL- | 0,00 | 1 |
| | Lavage Broncho Alvéolaire | PVL- | 0,00 | 1 |
| | Crachat | PVL- | 0,00 | 1 |
| | Lavage Broncho Alvéolaire | PVL- | 0,00 | ND |
| | Aspiration bronchique | PVL- | 0,00 | 0 |
| | Aspiration bronchique | PVL- | 0,00 | 0 |
| | Aspiration bronchique | PVL- | 0,00 | 0 |
| | Lavage Broncho Alvéolaire | PVL- | 0,00 | 0 |
| | Aspiration bronchique | PVL- | 0,00 | 1 |
| | Aspiration bronchique | PVL- | 0.00 | 0 |
| | Aspiration bronchique | PVL- | 0,00 | 1 |
| | Aspiration bronchique | PVL- | 0,00 | 0 |
| | Aspiration bronchique | PVL- | 0,00 | 0 |
| | Crachat | PVL- | 0,00 | 0 |
| | Aspiration bronchique | PVL- | 0,00 | 0 |
| | Aspiration bronchique | PVL- | 0,00 | 0 |
| | Aspiration bronchique | PVL- | 0,00 | ND |
| | Aspiration bronchique | PVL- | 0,00 | 1 |
| | Aspiration bronchique | PVL- | 0,00 | 0 |
| Infection de la peau | Abcès | PVL+ | > 1 | 0 |
| | Abcès | PVL+ | 0.42 | 0 |
| | Abcès | PVL+ | >1 | 0 |
| | Abcès | PVL+ | >1 | 0 |
| | Abcès | PVL+ | 1,20 | 0 |
| | Abcès | PVL+ | 1,00 | 0 |
| | Abcès | PVL+ | 1,30 | 0 |
| | Abcès | PVL+ | 1,50 | 1 |
| | Abcès | PVL+ | 1,82 | 0 |
| | Abcès | PVL+ | >2 | 0 |
| | Abcès | PVL+ | 0,92 | 0 |
| | Abcès | PVL+ | >2 | 0 |
| | Abcès | PVL+ | 0,27 | 0 |
| | Abcès | PVL+ | >2 | 0 |
| | Abcès | PVL+ | 1,72 | 0 |
| | Abcès | PVL+ | 1,41 | 0 |
| | Abcès | PVL- | 0,00 | 0 |
| | Abcès | PVL- | 0,00 | 1 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0.00 | 0 |
| | Abcès | PVL- | 0.00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 1 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |
| | Abcès | PVL- | 0,00 | 0 |

| | | | | |
|---|---|---|---|---|
| * L'appartenance à PVL- ou PVL+ a été mise en oeuvre par la détection de la présence par PCR des gènes lukS-PV et lukF-PV codant la PVL au sein des isolats de *S aureus* selon les méthodes décrites par Vandenesch et al.² ** La détection du gène mecA codant la résistance à la méthicilline au sein des isolats de *S aureus* a été mise en oeuvre selon les méthodes décrites par Vandenesch et al.² | | | | |

Bibliographie
1 : Ward PD, Turner WD, 1980, Infect. Immun., 28(2) : 393-397
2 : Vandenesch et al.. 2003, Emerg Infect Dis, 9(8) : 978-984
3 : Dufour P et al., 2002, Clin Infect Dis, 35 : 819-824
4 : Cribier B et al. 1992, Dermatology, 185 : 175-185
5 : Freney J et al., 2000, Précis de Bactériologie Clinique, 40 : 298 et 793-794
6 : J. Histochem, Cytochem. 45 : 481-491, 1997
7 : Labandeira-Rey M et al, Science 2007, sous presse

## Revendications

1. Procédé de diagnostic *in vitro* des *Staphylococcus aureus* producteurs de Panton Valentine Leukocidin (PVL) à partir d'un échantillon biologique issu d'un individu susceptible d'être colonisé ou infecté par des *Staphylococcus aureus,* le diagnostic étant mis en oeuvre par détection de PVL par un test immunologique de routine, **caractérisé en ce que** ledit échantillon biologique est préalablement traité pour dénaturer la PVL.

2. Procédé selon la revendication 1, **caractérisé en ce que** le test immunologique de routine est une méthode sandwich.

3. Procédé selon la revendication 1 ou 2, **caractérisé en ce que** le test immunologique met en oeuvre au moins un anticorps monoclonal anti-PVL.

4. procédé selon la revendication 2 ou 3, **caractérisé en ce que** la méthode sandwich est un test ELISA

5. Procédé selon l'une quelconque des revendications 1 à 4, **caractérisé en ce que** le traitement préalable consiste en un chauffage à une température comprise entre 60 et 100 °C en une durée d'au moins 10 min.

6. Procédé selon l'une quelconque des revendications précédentes, **caractérisé en ce que** le traitement préalable consiste en un chauffage à une température comprise entre 60 et 100 °C en une durée comprise entre 10 et 30 min.

7. Procédé selon l'une quelconque des revendications 1 à 4, **caractérisé en ce que** le traitement préalable consiste en la dénaturation de la PVL- par un acide,

8. Procédé selon la revendication 7, **caractérisé en ce que** l'acide est KH₂PO₄.

9. Procédé selon l'une quelconque des revendications 1 à 8, **caractérisé en ce qu'**il comprend une étape supplémentaire consistant à déterminer si les *Staphylococcus aureus* présentes dans ledit échantillon biologique sont des bactéries résistantes à la méthicilline (MRSA) ou sensibles à la méthicilline (MSSA).

10. Procédé de diagnostic *in vitro* des *Staphylococcus aureus* producteurs de Panton Valentine Leukocidin (PVL) à partir d'un échantillon biologique issu d'un individu susceptible d'être colonisé ou infecté par des *Staphylococcus aureus,* le procédé comprenant les étapes de :
- prétraitement de l'échantillon pour dénaturer la PVL
- détection de la PVL par un test sandwich.

11. Procédé selon la revendication 10, **caractérisé en ce que** le prétraitement de l'échantillon est mis en oeuvre par un chauffage à une température comprise entre 60 et 100 °C en une durée d'au moins 10 min.

12. Procédé selon la revendication 10. **caractérisé en ce que** le prétraitement de l'échantillon est mis en oeuvre par un traitement par un acide.

13. Procédé de diagnostic *in vitro* des *Staphylococcus aureus* producteurs de Panton Valentine Leukocidin (PVL) à partir d'un échantillon biologique issu d'un individu susceptible d'être colonisé ou infecté par des *Staphylococcus aureus,* le procédé comprenant les étapes de :
- prétraitement de l'échantillon par un chauffage à une température comprise entre 60 et 100 °C en une durée d'au moins 10 min pour dénaturer la PVL
- détection de la PVL- par un test immunologique de routine.

14. Procédé de diagnostic *in vitro* des *Staphylococcus aureus* producteurs de Panton Valentine Leukocidin (PVL) à partir d'un échantillon biologique issu d'un individu susceptible d'être colonisé ou infecté par des *Slaphylococcus aureus,* le procédé comprenant les étapes de :
- prétraitement de l'échantillon par un l'utilisation d'un acide,
- détection de la PVL par un test immunologique de routine.


SEQUENCE LISTING

<110>  bioMérieux
       Institut National de la Santé et de la Recherche Médicale
       Université Claude Bernard Lyon 1

<120>  Procédé de diagnostic in vitro des Staphylococcus aureus
       producteurs de PVL

<130>  PVL STAPH

<160>  4

<170>  PatentIn version 3.3

<210>  1
<211>  309
<212>  PRT
<213>  Staphylococcus aureus

<400>  1

Met Ser Gly Ser His His His His His His Ser Ser Gly Ile Glu Gly
1               5                   10                  15

Arg Gly Arg Leu Ile Lys Ser Lys Ala Asp Asn Asn Ile Glu Asn Ile
            20                  25                  30

Gly Asp Gly Ala Glu Val Val Lys Arg Thr Glu Asp Thr Ser Ser Asp
        35                  40                  45

Lys Trp Gly Val Thr Gln Asn Ile Gln Phe Asp Phe Val Lys Asp Lys
    50                  55                  60

Lys Tyr Asn Lys Asp Ala Leu Ile Leu Lys Met Gln Gly Phe Ile Asn
65                  70                  75                  80

Ser Lys Thr Thr Tyr Tyr Asn Tyr Lys Asn Thr Asp His Ile Lys Ala
                85                  90                  95

Met Arg Trp Pro Phe Gln Tyr Asn Ile Gly Leu Lys Thr Asn Asp Pro
            100                 105                 110

Asn Val Asp Leu Ile Asn Tyr Leu Pro Lys Asn Lys Ile Asp Ser Val
        115                 120                 125

Asn Val Ser Gln Thr Leu Gly Tyr Asn Ile Gly Gly Asn Phe Asn Ser
    130                 135                 140

Gly Pro Ser Thr Gly Gly Asn Gly Ser Phe Asn Tyr Ser Lys Thr Ile
145                 150                 155                 160

Ser Tyr Asn Gln Gln Asn Tyr Ile Ser Glu Val Glu His Gln Asn Ser
                165                 170                 175

Lys Ser Val Gln Trp Gly Ile Lys Ala Asn Ser Phe Ile Thr Ser Leu
            180                 185                 190

Gly Lys Met Ser Gly His Asp Pro Asn Leu Phe Val Gly Tyr Lys Pro
        195                 200                 205

Tyr Ser Gln Asn Pro Arg Asp Tyr Phe Val Pro Asp Asn Glu Leu Pro
    210                 215                 220

Pro Leu Val His Ser Gly Phe Asn Pro Ser Phe Ile Ala Thr Val Ser
225                 230                 235                 240

His Glu Lys Gly Ser Gly Asp Thr Ser Glu Phe Glu Ile Thr Tyr Gly
                245                 250                 255

Arg Asn Met Asp Val Thr His Ala Thr Arg Arg Thr Thr His Tyr Gly
            260                 265                 270

Asn Ser Tyr Leu Glu Gly Ser Arg Ile His Asn Ala Phe Val Asn Arg
        275                 280                 285

Asn Tyr Thr Val Lys Tyr Glu Val Asn Trp Lys Thr His Glu Ile Lys
    290                 295                 300

Val Lys Gly His Asn
305

<210>  2
<211>  323
<212>  PRT
<213>  Staphylococcus aureus

<400>  2

Met Ser Gly Ser His His His His His His Ser Ser Gly Ile Glu Gly
1               5                   10                  15

Arg Gly Arg Leu Ile Lys Ala Gln His Ile Thr Pro Val Ser Glu Lys
            20                  25                  30

Lys Val Asp Asp Lys Ile Thr Leu Tyr Lys Thr Thr Ala Thr Ser Asp
        35                  40                  45

Ser Asp Lys Leu Lys Ile Ser Gln Ile Leu Thr Phe Asn Phe Ile Lys
    50                  55                  60

Asp Lys Ser Tyr Asp Lys Asp Thr Leu Ile Leu Lys Ala Ala Gly Asn
65                  70                  75                  80

Ile Tyr Ser Gly Tyr Thr Lys Pro Asn Pro Lys Asp Thr Ile Ser Ser

                85                  90                  95

Gln Phe Tyr Trp Gly Ser Lys Tyr Asn Ile Ser Ile Asn Ser Asp Ser
            100                 105                 110

Asn Asp Ser Val Asn Val Val Asp Tyr Ala Pro Lys Asn Gln Asn Glu
        115                 120                 125

Glu Phe Gln Val Gln Gln Thr Val Gly Tyr Ser Tyr Gly Gly Asp Ile
    130                 135                 140

Asn Ile Ser Asn Gly Leu Ser Gly Gly Gly Asn Gly Ser Lys Ser Phe
145                 150                 155                 160

Ser Glu Thr Ile Asn Tyr Lys Gln Glu Ser Tyr Arg Thr Ser Leu Asp
                165                 170                 175

Lys Arg Thr Asn Phe Lys Lys Ile Gly Trp Asp Val Glu Ala His Lys
            180                 185                 190

Ile Met Asn Asn Gly Trp Gly Pro Tyr Gly Arg Asp Ser Tyr His Ser
        195                 200                 205

Thr Tyr Gly Asn Glu Met Phe Leu Gly Ser Arg Gln Ser Asn Leu Asn
    210                 215                 220

Ala Gly Gln Asn Phe Leu Glu Tyr His Lys Met Pro Val Leu Ser Arg
225                 230                 235                 240

Gly Asn Phe Asn Pro Glu Phe Ile Gly Val Leu Ser Arg Lys Gln Asn
                245                 250                 255

Ala Ala Lys Lys Ser Lys Ile Thr Val Thr Tyr Gln Arg Glu Met Asp
            260                 265                 270

Arg Tyr Thr Asn Phe Trp Asn Gln Leu His Trp Ile Gly Asn Asn Tyr
        275                 280                 285

Lys Asp Glu Asn Arg Ala Thr His Thr Ser Ile Tyr Glu Val Asp Trp
    290                 295                 300

Glu Asn His Thr Val Lys Leu Ile Asp Thr Gln Ser Lys Glu Lys Asn
305                 310                 315                 320

Pro Met Ser

<210> 3
<211> 18

<212>  PRT
<213>  Staphylococcus aureus

<400>  3

Cys Ser Gly His Asp Pro Asn Leu Phe Val Gly Tyr Lys Pro Tyr Ser
1               5                   10                  15

Gln Asn

<210>  4
<211>  20
<212>  PRT
<213>  Staphylococcus aureus

<400>  4

Cys Asn Phe Asn Trp Ile Gly Asn Asn Tyr Lys Asp Glu Asn Arg Ala
1               5                   10                  15

Thr His Thr Ser
            20